# EUROPEAN PATENT APPLICATION

(11) **EP 4 235 201 A1**
(43) Date of publication of application: **30.08.2023**
(21) Application number: 22158588.8
(22) Date of filing: 24.02.2022
(51) Int. Cl.: G01R 33/54, G01R 33/56, G01R 33/563, G01R 33/567, A61B 6/00

(54) **COMPUTER-IMPLEMENTED METHOD FOR PARAMETRIZING A MAGNETIC RESONANCE MEASUREMENT SEQUENCE OR SUPPORTING THE PLANNING OF THE MAGNETIC RESONANCE MEASUREMENT SEQUENCE, MEDICAL IMAGING DEVICE, COMPUTER PROGRAM AND COMPUTER-READABLE STORAGE MEDIUM**

(71) Applicant: Siemens Healthcare GmbH, 91052 Erlangen (DE)
(72) Inventor: Stich, Manuel, 92711 Parkstein (DE); Rupp, Dominik, 90587 Tuchenbach (DE)

(57) **Abstract**

Computer-implemented method for either parametrizing a measurement sequence (49) or for determining output data to support the planning of the measurement sequence (49), wherein the measurement sequence (49) comprises an acquisition of image data at multiple positions (4, 5, 6), comprising the steps of:
- repeatedly acquiring a respective primary image data set (14) while the table (7) is arranged in a primary position (4) until a primary trigger condition (16) is fulfilled, wherein a primary timing information (17) concerning the time of the acquisition of the primary image data set (14) for which the primary trigger condition (16) was fulfilled is registered,
- using an actuator (13) to change the position (4, 5, 6) after the primary trigger condition (16) was fulfilled,
- repeatedly acquiring a respective secondary image data set (18) while the table (7) is positioned in the secondary position (6),
- on the one hand evaluating a secondary trigger condition (19, 20) and registering a secondary timing information (21, 22) and/or on the other hand registering a respective temporal evolution of an image property (15) in the sequence of the secondary image data sets (18), and
- determining at least one parameter value (23) to parametrize the measurement sequence (49) and/or the output data based on the primary timing information (17) and on the secondary timing information (21, 22) and/or the registered temporal evolution.

## Description

The invention concerns a computer-implemented method for either parametrizing a measurement sequence for controlling a medical imaging device for acquiring medical image data for a given patient or for determining output data that is output to a user to support the planning of the measurement sequence, wherein the measurement sequence comprises an acquisition of image data at multiple positions of the table carrying the patient along a given path with respect to an acquisition component. Additionally, the invention concerns a medical imaging device, a computer program and a computer-readable storage medium.

For some medical imaging procedures, e.g. for magnetic resonance angiography, the use of a contrast agent that can be injected prior to the imaging procedure is advantageous. In some of the imaging procedures, it is advantageous to measure rather large sections of the patient. It is e.g. common in peripheral magnetic resonance angiography to image an area starting at the abdomen of the patient down to the lower legs or even to the feet. At the same time, it can be advantageous to use a relatively short scanner that can only acquire image data over a length of e.g. 30 cm or 40 cm at the same time. The use of a short scanner can, e.g., reduce the cost and help to avoid claustrophobia of the patient. The use of a short scanner requires either the acquisition of several separate images in a sequence or a continuous movement of the table with respect to the scanner as e.g. suggested in the document US 2010/194390 A1.

Measurement sequences using a contrast agent and a relative movement of the table with respect to the acquisition component, e.g. an MR-scanner, are currently planned in such a way that an initial time for the contrast agent to reach a first measurement region, e.g. the abdomen, is determined either by using a small test-bolus of contrast agent of by recognizing the inflow of the contrast agent into this region on the fly. Once the inflow is detected or at the time at which the inflow is predicted, the measurement sequence is started and measurements at each measurement position are typically performed as fast as possible to ensure that the next measurement position is reached in time. Especially when modern approaches like compressed sensing are used to reduce the scan time, this approach can lead to unnecessary short acquisition times for the individual images or for sections of the patient and therefore to a suboptimal image quality.

These problems can in principle be avoided by not using any contrast agent as e.g. described in the documents US 2012/271158 A1 and US 2011/251477 A1. In many cases, the use of a contrast agent is however advantageous to achieve optimum image quality. The document US 2016/274782 A1 discusses scanning protocols that are intended to be used independently of the body region that is mapped.

The problem to be solved is therefore to provide a computer-implemented method that allows an improvement of the image quality when using a contrast agent.

The problem is solved by the computer-implemented method discussed above, comprising the steps:
- repeatedly acquiring a respective primary image data set using the acquisition component while the table is arranged in a primary position along the path until a primary trigger condition is fulfilled, wherein the fulfilment of the primary trigger condition depends on the most recently acquired primary image data set, wherein a primary timing information concerning the time of the acquisition of the primary image data set for which the primary trigger condition was fulfilled is registered,
- using an actuator to change the position of the table with respect to the acquisition component to a secondary position along the path, after the primary trigger condition was fulfilled,
- repeatedly acquiring a respective secondary image data set using the acquisition component while the table is positioned in the secondary position,
- on the one hand evaluating a secondary trigger condition that depends on the most recently acquired secondary image data set and registering a secondary timing information concerning the time of the acquisition of the secondary image data set for which the secondary trigger condition was fulfilled, and/or on the other hand registering a respective temporal evolution of an image property for at least one monitored region of interest in the sequence of the secondary image data sets, and
- determining at least one parameter value to parametrize the measurement sequence and/or the output data based on the primary timing information and on the secondary timing information and/or the registered temporal evolution.

The invention uses a preparatory acquisition of primary and secondary image data sets to determine timing information and/or a temporal evolution of an image property that is preferably indicative of an inflow of the contrast agent to allow a user to take the required time for a contrast agent distribution into account while planning the measurement sequence or to automatically parametrize the measurement sequence to take this time into account. If e.g. a relatively long time passes between the fulfillment of the primary and secondary trigger condition or between the primary trigger condition and the time at which the temporal evolution of the image property indicates a sufficient presence of contrast agent in a region of the patient imaged in the secondary position, the part of the measurement sequence prior to the image acquisition at the secondary position can be performed relatively slow and therefore a high image quality can be reached. On the other hand, a fast spread of the contrast agent to the region imaged at the secondary position can be taken into account by parametrizing the measurement sequence to speed up the part prior to the image acquisition at the secondary position.

The acquisition component can be a magnetic resonance scanner. Alternatively, the acquisition component could e.g. be an X-ray detector, e.g. in a computer tomograph, a PET-scanner, etc. The measurement sequence can provide a classic subtraction angiography in which imaging is performed sequentially first without contrast agent and then with contrast agent. Alternatively, e.g. a TWIST-sequence can be used in which time resolved measurements are performed while the relevant area is flooded with contrast agent.

The path can define a sequence of relative positions between the table and the acquisition component. While typically the position is varied along the path by moving the table with respect to the acquisition component, it would alternatively also be possible to move the acquisition component with respect to the table to change the position.

The at least one parameter can be provided via an interface to the medical imaging device or another device that is determining and/or controlling the measurement sequence. Alternatively, the computer-implemented method itself can be performed by the medical imaging device to internally parametrize the measurement sequence. Output information can be provided via an output interface, e.g. to a display device, a printer or to a storage medium.

The image property can especially be a brightness and/or a contrast in the monitored region. Preferably, the image quality or certain values of the image quality can be indicative of the presence of contrast agent in the monitored region of interest.

The fulfillment of the trigger condition can especially depend on the presence of contrast agent in the section of the patient for which the respective image data set was acquired or in at least one region of interest in this section.

To achieve a high temporal resolution for the respective timing information and/or the registered temporal evolution of the image property, it advantageous to use a relatively fast acquisition protocol for acquiring the resspective image data set. It is e.g. possible to only sample the center of the k-space or to use sparse imaging techniques.

The temporal evolution of the image property can be registered for several monitored regions of interest in the secondary image data set. It is e.g. possible to use a separate monitored region of interest in each leg of a patient.

The discussed method is not limited to the acquisition of respective image data sets in the primary and secondary position. It is e.g. possible to move to a third position along the path after the secondary trigger condition is fulfilled. In this case it is possible that the secondary position is positioned between the primary and tertiary position along the path. The primary position can e.g. allow an imaging of the abdomen of the patient, the secondary position an imaging of the upper legs and the tertiary position an imaging of the lower legs and/or feet.

Alternatively, the primary position can lie between the secondary and tertiary position along the path. It is e.g. possible to image the area of the heart in the primary position, then image the area of the head in the secondary position and then image the legs in the tertiary position.

In the tertiary position, a tertiary timing information can be determined and the parameter value or the output data can additionally depend on this tertiary timing information. Alternatively or additionally, the temporal evolution of an image property of at least one monitored region can be registered for the tertiary image data set recorded at the tertiary position and the parameter value and/or output data can depend on this temporal evolution.

This approach could in principle be further extended to include four or more positions in which data sets are recorded to determine respective timing information and/or a respective temporal evolution of an image property.

The temporal extension of the measurement sequence and/or of at least one given section of the measurement sequence can depend on the determined parameter value. The determined parameter value can e.g. be a ramping speed of at least one field gradient, a sample density in k-space, the value of a parameter of a sparse sensing protocol, etc. The parameter can especially concern a section of the sequence that begins at or after the starting point of the sequence and that ends when or before the relative position of the table reaches the secondary position within the measurement sequence. In other words, the parametrization of the measurement sequence can serve to speed up or slow down measurements in the part of the measurement sequence prior to the image acquisition at the secondary position and therefore optimize image quality for this section of the measurement sequence given the constraints due to the use of a contrast agent.

At least one further timing information can be determined based on the primary and secondary timing information and at least one given property of the given patient, wherein the parameter value or of at least one of the parameter values depends on the further timing information. As discussed in more detail below, the further timing information can especially concern a time at which a measurement at a further position of the table with respect to the acquisition component should be started in the measurement sequence.

The further timing information can be determined by using a multi-parameter flow model of the transport of a contrast agent in the vascular system of the patient, that can especially describe the local flow velocity. It is e.g. possible to use a relatively simple model, that assigns different flow speeds to different vessels or to different positions of the patient in a direction parallel to the path, especially in the direction of the height of the patient.

The further timing information can be associated with a further position along the path, wherein the further timing information describes an expected time of arrival of a contrast agent in a section of the patient for which image data can be acquired by the acquisition component in the further position or in a specific region of interest in this section. The determination of the further timing information based on the primary and secondary timing information and the property or properties of the given patient can therefore be used instead of a determination of a further timing information based on an acquisition of a further image data set in the further position and the evaluation of a further trigger condition to determine this timing information. It therefore allows for a determination of more detailed timing constraints for the measurement sequence without the need to acquire additional data. This can especially be advantageous, since limitations in the measurement and movement speed of the acquisition component and table can limit the number of pieces of timing information that can be acquired based on the acquisition of respective image data sets for a respective position.

The further timing information can especially be used to determine parameter values for parameters of the measurement sequence concerning multiple sections of the measurement sequence. In an example where the further position is located along the path between the primary and the secondary position, a respective parameter value for at least one parameter influencing the temporal extension of a section of the measurement sequence prior to the image acquisition at the further position can be determined based on the primary and further timing information and a respective parameter value for at least one parameter parametrizing a section of the measurement sequence starting with the acquisition of image data at the further position can be determined based on the further and secondary timing information.

The property or at least one of the properties of the patient used to determine the further timing information can be selected from the group: age, height, weight and gender. Such properties can e.g. be extracted from a patient data record. The mentioned properties were found to be especially relevant when parametrizing a model of the blood flow and therefore the flow of a contrast agent in the vascular system.

The further timing information can describe a time between the time described by the primary timing information and the time described by the secondary timing information. Alternatively or preferably additionally, the further position can be positioned along the path between the primary and secondary position. It was found that under these conditions the further timing information can predict the arrival of the contrast agent in the section of the patient imaged in the further position with high accuracy.

The fulfillment of the primary trigger condition can depend on the image property or a further image property in a primary region of interest in the most recently acquired primary image data set. Additionally or alternatively, the fulfillment of the secondary trigger condition can depend on the image property or the further image property in the monitored region of interest or at least one of the monitored regions of interest and/or in a secondary region of interest in the most recently acquired secondary image data set. In general, the fulfillment of the respective trigger condition can therefore depend on an image property, e.g. a brightness or a contrast, in a respective region of interest in the respective image data set. The evaluated image property can especially be indicative of the presence of a contrast agent in the respective region of interest.

When the temporal evolution of a certain image property is registered for a monitored region, the same image property can be evaluated by the trigger conditions or a different image property can be used. It is e.g. possible to register the temporal evolution of the brightness in the monitored region and to evaluate a contrast in this region or a different region in the respective trigger condition.

Two distinct secondary trigger conditions that depend on the most recently acquired secondary image data set can be evaluated, wherein the fulfillment of each of the secondary trigger conditions depends on the image property or the further image property in a respective one of the monitored or secondary regions of interest, wherein a respective secondary timing information concerning the time of the acquisition of the secondary image data set for which the respective secondary trigger condition was fulfilled is registered, wherein the parameter value depends on both pieces of secondary timing information.

The separate regions of interest can e.g. be located in separate legs or arms of the patient. The evaluation of separate secondary trigger conditions for the separate regions of interest can be advantageous to determine timing information concerning the arrival of contrast agent in the separate regions of interest and therefore e.g. in different legs or arms of the patient. Since an image acquisition at the secondary position should be performed while a contrast agent is present e.g. in both arms or legs located in the imaged section, the determination of two pieces of secondary timing information can further improve the knowledge about the timing of the contrast agent distribution in the patient body and therefore the timing of the planned or parametrized measurement sequence.

A respective further timing information can be determined for each of the two pieces of secondary timing information, wherein the respective further timing information is determined based on the primary and the respective secondary timing information and at least one given property of the given patient, wherein the parameter value or at least one of the parameter values of the measurement sequence depends on both pieces of further timing information. It is e.g. possible that the two pieces of secondary timing information concern regions of interest in the lower part of a respective leg of the patient. The respective piece of further timing information can then concern an expected arrival time of contrast agent in a certain region of the upper part of the respective leg of the patient and therefore a time after which an imaging of the respective upper leg is desirable.

The inventive method can be used as part of a medical imaging workflow, especially of a magnetic resonance angiography workflow. A small dose, e.g. one milliliter, of a contrast agent can be introduced into the patient body. After this preparatory step the inventive method can be used to determine respective timing information or a temporal evolution of image properties concerning the arrival or concentration of contrast agent in certain regions of the body. This information can be processed by the inventive method to automatically parametrize a measurement sequence for a later measurement or to provide output data to a user to support the user in planning this measurement sequence.

The measurement sequence itself can preferably be performed after a larger dose of contrast agent is introduced into the patient body, preferably in the same way that the smaller dose was introduced, therefore leading to the same or at least similar time until certain body regions are reached by the contrast agent. Due to the parametrization of the measurement sequence by the inventive method, parts of the measurement sequence prior to an image acquisition at a certain position can be performed fast enough to ensure that this position is reached in time to allow for an imaging using contrast agent, while at the time providing the maximum measuring time and therefore image quality prior to reaching this position.

Besides the inventive method the invention also concerns a medical imaging device comprising a table for carrying a patient, an acquisition component for acquiring medical image data concerning the patient, an actuator to change the position of the table with respect to the acquisition component and a control unit for controlling the actuator and the acquisition component, wherein the control unit is adapted to implement the computer-implemented method according to the present invention. Features discussed with reference to the computer-implemented method according to the present invention can be transferred to the medical imaging device according to the present invention with the discussed advantages and vice versa.

Preferably, the control unit is also adapted to control the actuator and the acquisition component to execute the planned or parametrized measurement sequence.

The medical imaging device can comprise or control a component for introducing contrast agent into the patient body or be connected with such a component to e.g. receive a trigger signal or other timing information concerning the time of the introduction of contrast agent. It is therefore e.g. possible to reference the respective timing information and/or temporal evolution of the image property determined in the computer-implemented method discussed above to the time of a first contrast agent introduction and to reference the timing of the measurement sequence itself to the time of a second contrast agent introduction.

The invention also concerns a computer program comprising instructions to cause the medical imaging device according to the present invention to execute the computer-implemented method according to the present invention. It is e.g. possible to load the computer program into a memory of the control unit and to execute the program using a processor of a control unit.

Additionally, the invention concerns a computer-readable storage medium having stored thereon the computer program according to the present invention.

Other objects and features of the present invention will become apparent from the following detailed description considered in conjunction with the accompanying drawings. The drawings, however, are only principle sketches designed solely for the purpose of illustration and do not limit the invention. The drawings show:
- Fig. 1: an exemplary embodiment of a medical imaging device according to the present invention,
- Fig. 2: a flowchart of an exemplary embodiment of the computer-implemented method according to the present invention,
- Fig. 3: an illustration of imaged sections of a patient for various relative positions of the table carrying the patient to an acquisition component of the medical imaging device shown in fig. 1 and relevant regions of interest, and
- Fig. 4: an exemplary diagram showing approximate flow speeds of a contrast agent at different positions along a patient body.

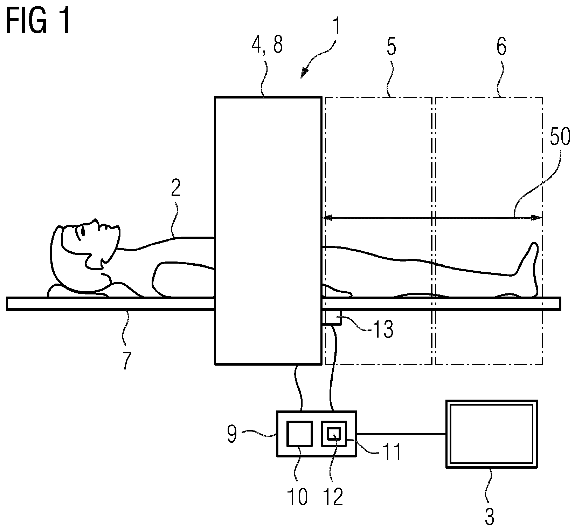

Fig. 1 shows a medical imaging device 1 comprising a table 7 for carrying the patient, an acquisition component 8 for acquiring medical image data for the patient, an actuator 13 to change the position of the table 7 with respect to the acquisition component 8 and a control unit 9 for controlling the actuator 13 and the acquisition component 8 during a measurement sequence.

In the example the acquisition component 8 is a magnetic resonance scanner and the measurement sequence can e.g. perform a peripheral magnetic resonance angiography. During such a measurement sequence it is typically desirable to acquire image data over an extended section of the patient reaching e.g. from the abdomen to the lower legs or feet. By changing the relative position between table 7 and acquisition component 8, it is still possible to use a relatively short scanner. Such a relative movement is typically implemented in the area of magnetic resonance tomography by using a fixed position of the acquisition component 8 and moving the table 7 using the actuator 13. For ease of visualization, fig. 1 shows a relative movement of the acquisition component 8 with respect to the table 7 and therefore the patient 2 instead. Obviously, both approaches can be used interchangeably.

For simplicity's sake the following discussion will assume that the measurement sequence comprises three separate acquisitions of image data in the relative positions 4, 5 and 6 between acquisition component 8 and table 7. The positions 4, 5, 6 are spaced apart along the path 50.

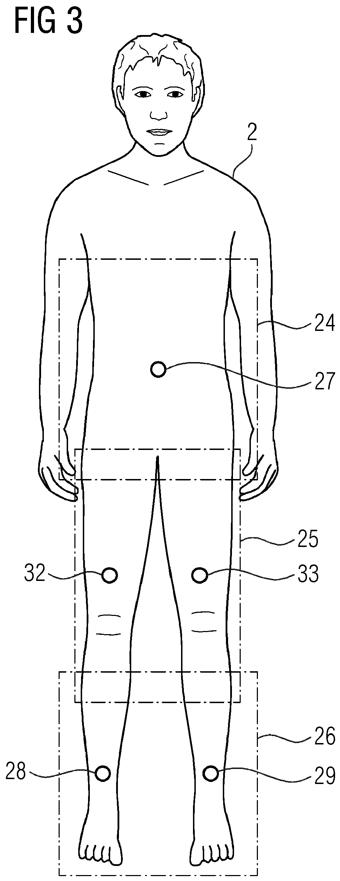

When a contrast agent is used during image acquisition, the image acquisition should be timed in such a way that the sections 24, 25, 26 of the patient shown in fig. 3 that can be depicted when the acquisition component 8 is in a respective position 4, 5, 6 with respect to the table 7, comprise a sufficient amount of contrast agent at the time of image acquisition to maximize the contrast. As an example, the contrast agent is introduced into the body of the patient 2 in such a way that at first the section 24 of the body of the patient 2 has a high contrast agent concentration and the contrast agent then spreads sequentially to the section 25 and the section 26.

In principle, the contrast could be maximized by performing the image acquisition in the position 4 as fast as possible, then move to the position 5, wait until a presence of the contrast agent is detected and then again perform a fast measurement at the position 5. After this fast measurement the position can be changed to the position 6 and a measurement in this position can be performed once a sufficient concentration of contrast agent is detected.

A drawback of this approach is the need to reduce the measurement time at the positions 4 and 5 to ensure, that the following positions 5, 6 are reached in time for any possible patient 2. The available measuring time at a given position 4, 5, 6 does however typically limit the image quality, since e.g. the use of sparse sampling techniques, a low resolution in k-space and very fast gradient sequences that can be used to speed up the measurement, typically reduce the image quality.

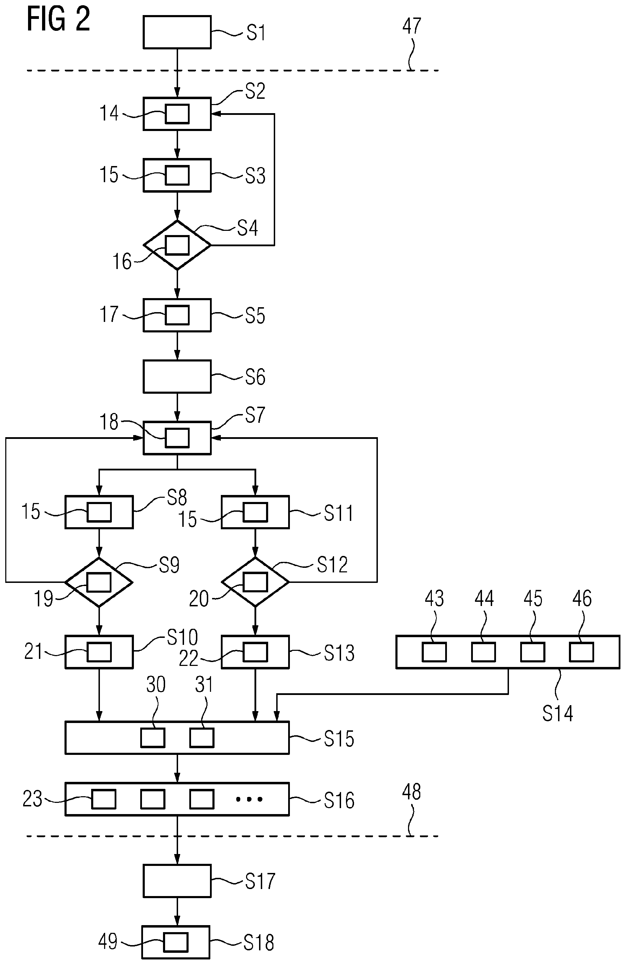

To avoid this problem, the medical imaging device 1 implements a computer-implemented method for parametrizing the measurement sequence 49 used for acquiring the medical image data for the patient 2 that is discussed with additional reference to fig. 2.

In the example, the control unit 9 comprises a processor 10 and a memory unit 11 storing a computer program 12 for controlling the acquisition component 8 and the actuator 13 for implementing the method. The computer-implemented method for parametrizing the measurement sequence 49 only comprises the steps S2 to S16. As indicated by the dashed lines 47, 48 the steps S1, S17 and S18 are additional steps that are not part of the method and are only discussed for context.

The core idea of the method is to determine various pieces of timing information 17, 21, 22, 30, 31 that describes when measurements in certain sections 24, 25, 26 of the patient 2 and therefore in different relative positions 4, 5, 6 between a table 7 and the acquisition component 8 can advantageously be performed, especially since a sufficient concentration of contrast agent in the respective section 24, 25, 26 is expected at the time described by the respective timing information 17, 21, 22, 30, 31.

In the example, a contrast agent is first introduced into the patient 2 in a preliminary step S1 prior to the execution of the computer-implemented method. Preferably, the time of the introduction of the contrast agent is recorded. For this purpose, it is e.g. possible that the control unit 9 triggers the introduction of the contrast agent at a given time or communicates with a device for introducing the contrast agent to receive timing information, e.g. a trigger signal from this device.

In step S2, a primary image data set 14 is acquired using the acquisition component 8 while the table is arranged in the primary position 4 along the path 50 with respect to the acquisition component 8. Therefore, this acquired image data concerns the section 24 of the patient 2 shown in fig. 3.

Steps S3 and S4 serve to evaluate a trigger condition 16 that depends on the most recently acquired primary image data set 14. If the trigger condition 16 is not fulfilled, step S2 is repeated to acquire a further primary image data set 14 until the most recently acquired primary image data set 14 fulfills the primary trigger condition 16.

In the example, the fulfillment of the primary trigger condition 16 in step S4 depends on an image property 15, e.g. the brightness or a contrast, in a primary region 27 of interest shown in fig. 3 in the most recently acquired primary image data set 14. It is e.g. possible to use previous whole body and/or localization scans to determine the position of the primary region 27 of interest with respect to the table 7 and therefore, due to the known position 4 of the table 7 with respect to the acquisition component 8, the acquisition component. The primary trigger condition can e.g. compare the determined image property 15 with a given threshold value.

Once the primary trigger condition 16 is fulfilled in step S4 for the most recently acquired primary image data set 14, a primary timing information can be recorded in step S5, that concerns the time at which the primary image data set 14 that fulfilled the primary trigger condition 16 was acquired.

In step S6, the control unit 9 controls the actuator 13 to change the relative position of the table 7 and the acquisition component 8 to a secondary position 6 to acquire the section 26 of the patient 2 comprising the lower legs and feet.

Once the position 6 is reached, a secondary image data set 18 can be acquired in step S7.

In the shown example, two separate trigger conditions 19, 20 that evaluate the most recently recorded secondary image data set 18 are evaluated and further processing is used in the following steps to estimate timing information for an image acquisition in the relative position 5 between table 7 and acquisition component 8. To more clearly illustrate the basic idea of the discussed method, at first a simplified alternate embodiment will be discussed, in which the steps S11 to S15 are skipped.

The steps S8 and S9 serve to evaluate a secondary trigger condition 19. The evaluation of the trigger condition 19 in step S9 is quite similar to the evaluation of the trigger condition 16 in step S4. The image quality 15 is determined in step S8 for the monitored region 28 of interest in the section 26 of the patient 2 depicted in the most recently acquired secondary image data set 18 and e.g. compared to a threshold in step S9.

While the secondary trigger condition 19 is not fulfilled, additional secondary image data sets 18 are acquired by repeating a step S7. Once the secondary trigger condition 19 is fulfilled, the secondary timing information 21 concerning the time of the acquisition of the secondary image data set 18 that fulfilled the secondary trigger condition 19 is recorded in step S10.

In the simplified embodiment that skips the steps S11 to S15, at least one parameter value 23 to parametrize the measurement sequence 49 is then determined in step S16 based on the primary and secondary timing information 17, 21. Since the primary timing information 17 describes the time at which a measurement at the primary position 4 provides a sufficient image quality due to the presence of contrast agent and the secondary timing information 21 describes this time for the position 6, the difference between the times given by the pieces of timing information 17, 21 essentially describes the time available for performing the image data acquisition at the positions 4 and 5 or more generally, e.g., when an image acquisition during a relative movement of table 7 and acquisition component 8 is used, for any image acquisitions before the position 6 is reached.

The temporal extension of a measurement sequence and especially of the section of the measurement sequence before the position 6 is reached can be scaled by modifying various parameters, e.g. a ramping speed of field gradients, a density of the k-space sampling and/or the use of sparse sampling techniques. Therefore, parameter values for these and other parameters influencing the timing of the measurement sequence can be automatically selected in step S16 to match the primary and secondary timing information 15, 21.

By parametrizing the measurement sequence in this way a sufficient concentration of contrast agent in the respective measured sections 24, 25, 26 can be ensured while at the same time avoiding very fast measurements that can lower the image quality.

As shown in fig. 3, the section 26 of the patient 2 imaged in the secondary position 6 comprises two separate legs. Depending on the vascular system of the imaged patient 2, the time required for a contrast agent to reach the respective lower leg can be quite different for the left and right leg. Therefore, it can be advantageous to use the additional steps S11 to S13 to determine a further secondary timing information 22. In step S11, the image quality 15 is determined for a further monitored region 29 of interest and therefore for the other leg of the patient 2.

In step S12 a separate secondary trigger condition 20 is evaluated for this region 29, e.g. by comparing the image quality 15 determined in step S11 to a threshold value. The acquisition of the secondary image data sets 18 in step S7 is repeated until both secondary trigger conditions 19, 20 are fulfilled. Once the secondary trigger condition 20 in step S12 is fulfilled, a further secondary timing information 22 concerning the time of the acquisition of the secondary image data set 18 that fulfilled the further secondary trigger condition 20 is recorded in step S13.

In principle, the steps S14 and S15 could still be skipped and the parameter values 23 of the measurement sequence 49 could be determined in step S16 based on the primary and secondary timing information 17, 21, 22. Since it is typically desirable to start imaging at the position 6 once a sufficient concentration of contrast agent is present in both legs, the section of the measurement sequence 49 that is performed before the secondary position 6 is reached, can be scaled to match the time difference between the time described by the primary timing information 16 and the later time of the times described by the pieces secondary timing information 19, 20. Relevant parameters for scaling the length of sections of the measurement sequence were already discussed.

To further optimize the timing of the measurement sequence 49, it is advantageous to determine a further timing information for the position 5 that describes an expected time of arrival of the contrast agent in the section 25 of the patient 2 for which image data can be acquired by the acquisition component 8 in this relative position 5 between table 7 and acquisition component 8 or e.g. at least one region 32, 33 of interest in this section 25. In principle, it would, e.g., be possible to perform an additional acquisition of image data sets in the position 5 once the primary trigger condition is fulfilled, wait for the fulfilment of a trigger condition for the position 5 and only then move on to the secondary position 6. This approach can however be tricky to implement due to limitations of the acquisition and movement speed, especially when timing information should be provided for more than the three positions 4, 5, 6 discussed in the example.

Therefore, the steps S14 and S15 implement a different approach for providing further timing information 30, 31 for the further position 5. Step S15 implements a flow model for determining the further timing information 30, 31 and step S14 parametrizes this model by providing properties of the respective patient 2, e.g. an age 43, a height 44, a weight 45 and/or a gender 46. These properties of the patient 2 can e.g. be provided by a patient data record.

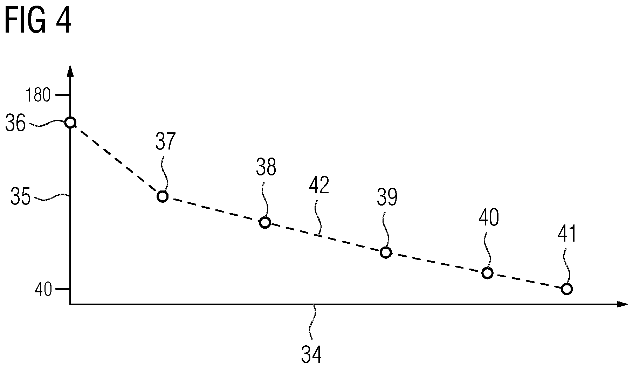

Fig. 4 shows an example describing the correlation of an expected flow velocity 35 given in cm/second with a position 34 along the height direction of the patient 2 for given values of the patient properties given in step S14. The properties of the patient 2 given in step S14 can e.g. select between different curves 42 or parametrize the given curve 42. The shown curve 42 is determined by determining a respective flow velocity for different vessels, in the example the thorical aorta 36, the iliac arteries 37, the femoral artery 38, the popliteal artery 39, the tibial artery 40 and the peroneal artery 41, and by assuming a linear change of velocity between the known velocity points. The velocities for the different vessels can e.g. be taken from literature.

In principle, the flow velocities given by fig. 4 could directly be used to calculate an expected time for the contrast agent to spread e.g. from the region 27 of interest to a region 32 or 33 of interest and therefore calculate the timing information 30, 31 for the different legs of the patient 2 based exclusively on the primary timing information 15. This approach would however ignore changes of the flow speed due to unknown or not considered properties of the patient 2, e.g. of a presence of a high vessel calcification.

It is therefore advantageous to additionally consider the secondary timing information 21, 22. For example, the flow velocities given by fig. 4 can be used to calculate an expected time for the contrast agent to travel from the region 27 to the region 28. By dividing the difference between the time given by the primary timing information 17 and the time given by the secondary timing information 21 by the expected time, a scaling factor can be calculated. A scaling factor larger than 1 indicates a slower flow velocity than expected due to the model and a scaling factor smaller than 1 indicates a faster flow velocity than the flow velocity given by the model.

By multiplying the expected flow time from the region 27 to the region 32 by the scaling factor, a more accurate timing information 30 can be determined.

The same approach can be used to determine the timing information 31 based on the primary timing information 17 and the secondary timing information 22.

A measurement in the section 25 should be performed at a time when the contrast agent has reached both regions 32, 33 of interest. Therefore, the later time described by the pieces of further timing information 30, 31 is selected and a time difference to the time described by the primary timing information 17 is calculated to determine a time available for a section of the measurement sequence 49 performed before the position 5 is reached. This time can especially be used to determine parameter values for the measurement at the position 4.

The further timing information 30, 31 and secondary timing information 21, 22 can then be used to parametrize the section of the measurement sequence that involves the measurement in the position 5 or more generally any measurements and movements prior to reaching the position 6 once the position 5 is reached.

Once the parameter values 23 are determined in step S16, the measurement sequence 49 is completely parametrized. The parametrized measurement sequence 49 can especially be used in step S18 after additional contrast agent is introduced into the patient 2 in step S17.

It is especially possible that the control unit 9 is adapted to control the actuator 13 and the acquisition component 8 to perform the measurement according to the measurement sequence 49 in step S18. Optionally, the control unit 9 can also be adapted to control a device for introducing the contrast agent into the body of the patient 2 in a step S17 or to receive timing information or a trigger signal from such a device.

For parametrizing the last part of the measurement sequence, it can be useful to evaluate the temporal evolution of the image property 15 in at least one monitored region 28, 29 in the sequence of secondary imaging data sets 18. It is e.g. possible to continue acquiring secondary image data sets 18 even after the secondary trigger conditions 19, 20 are both fulfilled and to record the sequence of the respective acquired image property 15 for each region 28, 29 for all acquired secondary image data sets 18. This temporal evolution can then e.g. be evaluated to determine a time of maximum concentration of contrast agent or a time interval for which the amount of present contrast agent is above a certain threshold. This information can be used to determine parameter values used for the image acquisition at the secondary position 6.

The previously discussed method performed a completely automatic parametrization of the measurement sequence 49. Some users, e.g. some medical personal, might however prefer to plan the measurement sequence themselves while being supported in this planning by output data that can e.g. comprise the various timing information 17, 21, 22, 30, 31 and/or the discussed temporal evolution. Such output data can e.g. be displayed on an output device 3.

Although the present invention has been described in detail with reference to the preferred embodiment, the present invention is not limited by the disclosed examples from which the skilled person is able to derive other variations without departing from the scope of the invention.

## Claims

1. Computer-implemented method for either parametrizing a measurement sequence (49) for controlling a medical imaging device (1) for acquiring medical image data for a given patient (2) or for determining output data that is output to a user to support the planning of the measurement sequence (49), wherein the measurement sequence (49) comprises an acquisition of image data at multiple positions (4, 5, 6) of a table (7) carrying the patient (2) along a given path (50) with respect to an acquisition component (8), comprising the steps of:
- repeatedly acquiring a respective primary image data set (14) using the acquisition component (8) while the table (7) is arranged in a primary position (4) along the path (50) until a primary trigger condition (16) is fulfilled, wherein the fulfilment of the primary trigger condition (16) depends on the most recently acquired primary image data set (14), wherein a primary timing information (17) concerning the time of the acquisition of the primary image data set (14) for which the primary trigger condition (16) was fulfilled is registered,
- using an actuator (13) to change the position (4, 5, 6) of the table (7) with respect to the acquisition component (8) to a secondary position (6) along the path (50), after the primary trigger condition (16) was fulfilled,
- repeatedly acquiring a respective secondary image data set (18) using the acquisition component (8) while the table (7) is positioned in the secondary position (6),
- on the one hand evaluating a secondary trigger condition (19, 20) that depends on the most recently acquired secondary image data set (18) and registering a secondary timing information (21, 22) concerning the time of the acquisition of the secondary image data set (18) for which the secondary trigger condition (19, 20) was fulfilled, and/or on the other hand registering a respective temporal evolution of an image property (15) for at least one monitored region (28, 29) of interest in the sequence of the secondary image data sets (18), and
- determining at least one parameter value (23) to parametrize the measurement sequence (49) and/or the output data based on the primary timing information (17) and on the secondary timing information (21, 22) and/or the registered temporal evolution.

2. Computer-implemented method according to claim 1, **characterized in that** the temporal extension of the measurement sequence (49) and/or of at least one given section of the measurement sequence (49) depends on the determined parameter value (23).

3. Computer implemented method according to claim 1 or 2, **characterized in that** at least one further timing information (30, 31) is determined based on the primary and secondary timing information (17, 21, 22) and at least one given property of the given patient (2), wherein the parameter value (23) or at least one of the parameter values (23) depends on the further timing information (30, 31).

4. Computer-implemented method according to claim 3, **characterized in that** the further timing information (30, 31) is associated with a further position (5) along the path (50), wherein the further timing information (30, 31) describes an expected time of arrival of a contrast agent in a section (25) of the patient for which image data can be acquired by the acquisition component (8) in the further position (5) or in a specific region (32, 33) of interest in this section (25).

5. Computer-implemented method according to claim 3 or 4, **characterized in that** property or at least one of the properties of the patient (2) used to determine the further timing information (30, 31) is selected from the group: age (43), height (44), weight (45) and gender (46) .

6. Computer-implemented method according to one of the claims 3 to 5, **characterized in that** the further timing information (30, 31) describes a time between the time described by the primary timing information (17) and the time described by the secondary timing information (21, 22) and/or **in that** the further position (5) is positioned along the path (50) between the primary and secondary position (4, 6).

7. Computer-implemented method according to one of the preceding claims, **characterized in that** the fulfilment of the primary trigger condition (16) depends on the image property (15) or a further image property in a primary region (27) of interest in the most recently acquired primary image data set (14) and/or **in that** the fulfilment of the secondary trigger condition (19, 20) depends on the image property (15) or the further image property in the monitored region (28, 29) of interest or at least one of the monitored regions (28, 29) of interest and/or in a secondary region of interest in the most recently acquired secondary image data set (18).

8. Computer-implemented method according to claim 7, **characterized in that** two distinct secondary trigger conditions (19, 20) that depend on the most recently acquired secondary image data set (18) are evaluated, wherein the fulfillment of each of the secondary trigger conditions (21, 22) depends on the image property (15) or the further image property in a respective one of the monitored or secondary regions (28, 29) of interest, wherein a respective secondary timing information (21, 22) concerning the time of the acquisition of the secondary image data set (18) for which the respective secondary trigger condition (19, 20) was fulfilled is registered, wherein the parameter value (23) depends on both pieces of secondary timing information (21, 22).

9. Computer-implemented method according to claim 8, **characterized in that** a respective further timing information (30, 31) is determined for each of the two pieces of secondary timing information (21, 22), wherein the respective further timing information (30, 31) is determined based on the primary and the respective secondary timing information (17, 21, 22) and at least one given property of the given patient (2), wherein the parameter value (23) or at least one parameter value (23) of the measurement sequence depends on both pieces of further timing information (30, 31).

10. Medical imaging device comprising a table (7) for carrying a patient (2), an acquisition component (8) for acquiring medical image data concerning the patient (2), an actuator (13) to change the position (4, 5, 6) of the table (7) with respect to the acquisition component (8) and a control unit (9) for controlling the actuator (13) and the acquisition component (8), **characterized in that** the control unit (9) is adapted to implement the computer-implemented method of one of the preceding claims.

11. Medical imaging device according to claim 10, **characterized in that** the control unit (9) is also adapted to control the actuator (13) and the acquisition component (8) to execute the planned or parametrized measurement sequence (49).

12. Computer program comprising instructions to cause the medical imaging device (1) of claim 10 or 11 to execute the computer-implemented method according to one of the claims 1 to 9.

13. Computer-readable storage medium having stored thereon the computer program (12) according to claim 12.
